(19) Europäisches Patentamt
European Patent Office
Office européen des brevets

(11) **EP 3 968 964 B1**

(12) **EUROPEAN PATENT SPECIFICATION**

(45) Date of publication and mention
of the grant of the patent:
**10.07.2024 Bulletin 2024/28**

(21) Application number: **20726433.4**

(22) Date of filing: **18.05.2020**

(51) International Patent Classification (IPC):
*A61K 9/16* (2006.01)    *A61K 9/51* (2006.01)

(52) Cooperative Patent Classification (CPC):
**A61K 9/5161; A61K 9/16; A61K 9/51; A61K 9/5192**

(86) International application number:
**PCT/EP2020/063852**

(87) International publication number:
**WO 2020/229702 (19.11.2020 Gazette 2020/47)**

(54) **METHOD FOR PRODUCING NANOPARTICLES**

VERFAHREN ZUR HERSTELLUNG VON NANOPARTIKELN

PROCÉDÉ DE PRODUCTION DE NANOPARTICULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2019 DE 102019112956**

(43) Date of publication of application:
**23.03.2022 Bulletin 2022/12**

(73) Proprietor: **leon-nanodrugs GmbH**
**82152 Planegg (DE)**

(72) Inventors:
• **HORSTKOTTE, Elke**
**81245 Munich Bavaria (DE)**
• **MÜHLHÖLZL-ODÖRFER, Kathrin**
**80469 Munich Bavaria (DE)**
• **VOGLER, Julian**
**88074 Meckenbeuren Baden-Württemberg (DE)**

(74) Representative: **Synergy IP Group AG**
**Leonhardsgraben 52**
**Postfach**
**4001 Basel (CH)**

(56) References cited:
**WO-A1-2014/043208     WO-A2-2008/065506**

• **MAKOTO UYAMA ET AL: "Useful Modified Cellulose Polymers as New Emulsifiers of Cubosomes", LANGMUIR, vol. 25, no. 8, 21 April 2009 (2009-04-21), US, pages 4336 - 4338, XP055718627, ISSN: 0743-7463, DOI: 10.1021/la900386q**
• **ANONYMOUS: "AquaSolveTM hydroxypropylmethylcellulose acetate succinate. Physical and chemical properties handbook", 1 January 2016 (2016-01-01), pages 1 - 16, XP055718300, Retrieved from the Internet <URL:https://www.ashland.com/file_source/Ashland/Industries/Pharmaceutical/Links/PC-12624.6_AquaSolve_HPMCAS_Physical_Chemical_Properties.pdf> [retrieved on 20200727]**
• **CUI-SHUAN WU ET AL: "Effects of pH-sensitive nanoparticles prepared with different polymers on the distribution, adhesion and transition of Rhodamine 6G in the gut of rats", JOURNAL OF MICROENCAPSULATION., vol. 27, no. 3, 1 May 2010 (2010-05-01), GB, pages 205 - 217, XP055718742, ISSN: 0265-2048, DOI: 10.3109/02652040903059163**
• **JESSON GÉRALD ET AL: "Carbon dioxide-mediated generation of hybrid nanoparticles for improved bioavailability of protein kinase inhibitors", PHARMACEUTICAL RESEARCH, KLUWER ACADEMIC PUBLISHERS-PLENUM PUBLISHERS, NL, vol. 31, no. 3, 30 August 2013 (2013-08-30), pages 694 - 705, XP002740110, ISSN: 1573-904X, DOI: 10.1007/S11095-013-1191-4**

- DUONG NHAT NGUYEN ET AL: "One-step production of darunavir solid dispersion nanoparticles coated with enteric polymers using electrospraying : Coated solid dispersion nanoparticles", PHARMACEUTICAL AND CLINICAL RESEARCH, vol. 68, no. 5, 14 August 2015 (2015-08-14), GB, pages 625 - 633, XP055718751, ISSN: 0022-3573, DOI: 10.1111/jphp.12459
- DUARTE ÍRIS ET AL: "Production of nano-solid dispersions using a novel solvent-controlled precipitation process - Benchmarking their in vivo performance with an amorphous micro-sized solid dispersion produced by spray drying", EUROPEAN JOURNAL OF PHARMACEUTICAL SCIENCES, ELSEVIER, AMSTERDAM, NL, vol. 93, 9 August 2016 (2016-08-09), pages 203 - 214, XP029736390, ISSN: 0928-0987, DOI: 10.1016/J.EJPS.2016.08.011
- RALM G. RICARTE ET AL: "Direct Observation of Nanostructures during Aqueous Dissolution of Polymer/Drug Particles", MACROMOLECULES, vol. 50, no. 8, 13 April 2017 (2017-04-13), WASHINGTON, DC, UNITED STATES, pages 3143 - 3152, XP055718771, ISSN: 0024-9297, DOI: 10.1021/acs.macromol.7b00372
- YINGYUE ZHANG ET AL: "Design and Solidification of Fast-Releasing Clofazimine Nanoparticles for Treatment of Cryptosporidiosis", MOLECULAR PHARMACEUTICS, vol. 14, no. 10, 20 September 2017 (2017-09-20), US, pages 3480 - 3488, XP055718773, ISSN: 1543-8384, DOI: 10.1021/acs.molpharmaceut.7b00521
- JIE FENG ET AL: "Rapid Recovery of Clofazimine-Loaded Nanoparticles with Long-Term Storage Stability as Anti-Cryptosporidium Therapy", ACS APPLIED NANO MATERIALS, vol. 1, no. 5, 20 April 2018 (2018-04-20), pages 2184 - 2194, XP055718775, ISSN: 2574-0970, DOI: 10.1021/acsanm.8b00234
- UEDA KEISUKE ET AL: "Mechanistic elucidation of formation of drug-rich amorphous nanodroplets by dissolution of the solid dispersion formulation", INTERNATIONAL JOURNAL OF PHARMACEUTICS, vol. 561, 26 February 2019 (2019-02-26), pages 82 - 92, XP085642913, ISSN: 0378-5173, DOI: 10.1016/J.IJPHARM.2019.02.034

**Description**

The present invention is directed to a method for producing nanoparticles, which are based on hydroxypropyl methylcellulose acetate succinate (HPMCAS).

WO 2014/043208 A1 discloses dispersions of enzalutamide and different polymers, such as HPMCAS. WO 2014/043208 A1 teaches spray drying of solutions containing acetone, active ingredient, and HPMCAS in order to obtain such dispersions. The obtained dispersions have particle sizes in the micrometer range. The poorly water-soluble active ingredient is embedded in different polymeric matrices in order to improve bioavailability and solubility of the active ingredient. However, for each active ingredient, the preparation method of the dispersion and its composition must be newly developed, which is expensive and time consuming.

Makato Uyama et al., Langmuir 2009, 25 (8), 4336-4338, prepared nanoparticles containing cubic-phase-forming lipids using HPMCAS.

Iris Duarte et al., European Journal of Pharmaceutical Sciences, 2016, 93(10), 203-214 discloses a solvent controlled precipitation process based on microfluidization.

Thus, there remains a need to develop an improved method for producing dispersions of HPMCAS and poorly water-soluble active ingredients. In particular, it is desired to obtain a method for producing particles with a size in the nanometer range. The method shall be suitable for producing nanoparticles, which contain an active ingredient or to which an active ingredient can be coupled later. Preferably, the same method is suitable for preparing nanoparticles with different active ingredients using similar process parameters or even without having to substantially modify the preparation method. Preferably, the nanoparticles have a large surface area and a small particle size. The nanoparticles are preferably well suitable to improve bioavailability and dissolution characteristics of the active ingredient. In particular, nanoparticles with an immediate release of the active ingredient in the gastrointestinal tract, more preferably the intestine, are preferably provided.

At least one of these objectives is solved by the present invention.

In a first aspect, the present invention is directed to a method for producing nanoparticles comprising an active ingredient and hydroxypropyl methylcellulose acetate succinate (HPMCAS), the method comprising the steps of a) providing an organic solution of the active ingredient and the HPMCAS in an organic solvent, b) providing an aqueous solution having a pH of 3 to 9; c) mixing the organic solution with the aqueous solution such as to precipitate the nanoparticles; wherein the organic solution is provided in the form of a first fluid stream; the aqueous solution is provided in the form of a second fluid stream; and wherein the mixing includes directing the first and the second fluid stream to contact one another; and wherein each of the first and the second fluid stream is ejected from a nozzle, and wherein the fluid streams are directed such as to impinge on one another; and optionally, wherein the method comprises a further step d) or isolating and/or drying the nanoparticles.

The term "nanoparticles" denotes particles with a size in the range of 1 to 1000 nm, measured by Dynamic Light Scattering (e.g. using a Malvern Zetasizer ZS90 from Malvern Instruments Ltd.). Dynamic Light Scattering (DLS) measures Brownian motion and relates this to the size of the particles. Brownian motion is the random movement of particles due to the bombardment by the solvent molecules that surround them. The larger the particle or molecule, the slower the Brownian motion will be. Smaller particles are "kicked" further by the solvent molecules and move more rapidly. An accurately known temperature is necessary for DLS because knowledge of the viscosity is required (because the viscosity of a liquid is related to its temperature). In the present measurement, a temperature of 25°C is used. This temperature is kept constant during the measurement. The velocity of the Brownian motion is defined by the translational diffusion coefficient (D). The size of a particle is calculated from the translational diffusion coefficient by using the Stokes-Einstein equation;

$$d(H) = \frac{kT}{3\pi\eta D}$$

Where d(H) is the hydrodynamic diameter, D is the translational diffusion coefficient, k is the Boltzmann's constant, T is the absolute temperature, and $\eta$ is the viscosity. The diameter that is obtained by the Stokes-Einstein equation is the diameter of a sphere that has the same translational diffusion coefficient as the particle. The particle translational diffusion coefficient will depend not only on the size of the particle "core", but also on any surface structure that will affect the diffusion speed, as well as the concentration and type of ions in the medium.

Malvern Zetasizer series measure the speed at which the particles diffuse due to Brownian motion by determining the rate at which the intensity of scattered light fluctuates when detected using a suitable optical arrangement. In the Zetasizer Nano ZS90 series, the detector position is 90°. The z-average diameter, together with the polydispersity index (PDI), are preferably calculated from the cumulants analysis of the DLS measured intensity autocorrelation function as defined in ISO22412:2008. PDI is a dimensionless estimate of the width of the particle size distribution, scaled from 0 to 1. Within this disclosure, the term particle size refers to the z-average diameter.

HPMCAS refers to hypromellose acetate succinate, which is as such generally known to the person skilled in the art. HPMCAS is an excipient for gastroresistant or enteric oral drug formulations, and also for the formation of solid dispersions. In the present invention, and without wishing to be bound by theory, it is predominantly used as a matrix-forming carrier, and in some cases it may also be able to inhibit crystallization of the API from the dispersion matrix. In the present invention, HPMCAS with an average molecular weight of about 10,000 to about 500,000 Da, or from about 10,000 to about 400,000 Da, or from about 55,000 to 93,000 Da, respectively, is preferably used, measured by gel permeation chromatography using polyethylene oxide as a relative reference standard. Preferably, free acid content is smaller than 1 %. The degree of substitution at the cellulose backbone of the HPMCAS preferably is between 3 and 14 %, preferably 5 and 9 % or 6 and 10 %, with hydroxypropyl and between 12 and 35 %, preferably 15 and 31 %, with methoxyl (methyl). Further, the degree of substitution at the cellulose backbone of the HPMCAS preferably is

(i) between 3 and 12 %, preferably 5 and 9 %, with acetate, and between 10 and 25 %, preferably 14 and 18 %, with succinate (e.g. HPMCAS 716G), or

(ii) between 5 and 15 %, preferably 7 and 11 %, with acetate, and between 8 and 18 %, preferably 10 and 14 %, with succinate (e.g. HPMCAS 912G), or

(iii) between 8 and 18 %, preferably 10 and 14 %, with acetate, and between 2 and 10 %, preferably 4 and 8 %, with succinate (e.g. HPMCAS 126G).

Preferably, the viscosity of the HPMCAS is between 2 and 5 cP, preferably 2.4 and 3.6 cP, measured as a 2.0 wt.-% solution of the esterified cellulose ether in 0.43 wt.-% aqueous NaOH at 20 °C. The 2.0 % by weight solution of the esterified cellulose ether is prepared as described in "Hypromellose Acetate Succinate, United States Pharmacopia and National Formulary, NF 29, pp. 1548-1550", followed by an Ubbelohde viscosity measurement according to DIN 51562-1:1999-01 (January 1999).

Examples of commercially available HPMCAS in different grades for use according to the invention are AFFINSIOL™ HPMCAS (in particular HPMCAS 716G, 912G, and 126G) from company Dow chemical, AquaSolve HPMCAS in different grades from company Ashland, or AQOAT Hypromellose Acetate Succinate from company ShinEtsu.

An active ingredient, as used herein, is a biologically active compound or mixture of compounds that is useful as an agent for medical or diagnostic treatments. In this context, medical treatments include the prevention, management, control, or therapy of a medical condition or any symptom associated therewith. Active ingredients include so-called small molecules, i.e. chemically defined compounds typically having a molecular weight of up to approximately 1,500 Da, as well as so-called biologics, such as therapeutic proteins. In one of the preferred embodiments, the active ingredient is a small molecule. The expression "drug substance" may also be used for an active ingredient useful in medical treatments.

In a preferred embodiment, the active ingredient is a poorly soluble compound having a water solubility selected from the group consisting of sparingly soluble, slightly soluble, very slightly soluble, and practically insoluble. In this context, the terms "sparingly soluble", "slightly soluble", "very slightly soluble", and "practically insoluble" are defined by the volume (ml) of water needed to dissolve 1 g of active ingredient, which is 30 to 100 for "sparingly soluble", 100 to 1000 for "slightly soluble", 1000 to 10000 for "very slightly soluble", and > 10000 for "practically insoluble". In view of the fact that the impact of a low water solubility on the oral bioavailability of an active ingredient also depends on the drug dose, it is furthermore preferred that the active ingredient is selected from drug substances classified as class 2 compounds or class 4 compounds according to the Biopharmaceutical Classification System (BCS: Guidance for Industry: Waiver of In Vivo Bioavailability and Bioequivalence Studies for Immediate-Release Solid Oral Dosage Forms Based on a Biopharmaceutics Classification System. FDA (December 2017)) or to the Biopharmaceutical Drug Disposition Classification System (BDDCS: Leslie Z. Benet: Predicting Drug Disposition via Application of a Biopharmaceutics Drug Disposition Classification System. Basic Clin Pharmacol Toxicol. 2010 March ; 106(3): 162-167). In the BCS, class 2 compounds have low solubility and high permeability, whereas class 4 compounds have low solubility and low permeability, wherein low solubility means that (at least) the highest dose strength used in oral immediate release formulations is soluble in 250 ml or less of aqueous media over the pH range of 1 to 7.5. According to the BDDCS, class 2 compounds have low solubility and are extensively metabolized, whereas class 4 compounds have low solubility and are poorly metabolized. In other words, according to both BCS and BDDCS, class 2 and 4 compounds are those that exhibit a low aqueous solubility relative to their oral dose.

Thanks to the HPMCAS matrix, which leads to a small size and a large surface area of the obtained particles, the bioavailability and dissolution of poorly water-soluble active ingredients is improved. Thus, oral application and immediate release in the gastrointestinal tract, more preferably the intestine, of poorly water-soluble active ingredients is improved or enabled.

In a further preferred embodiment, the active ingredient is selected from the group consisting of active pharmaceutical ingredients displaying low aqueous solubility, e.g. amiodarone, atorvastatin, azithromycin, carbamazepine, carvedilol, chlorpromazine, cisapride, ciprofloxacin, cyclosporine, danazol, dapsone, diclofenac, diflunisal, digoxin, erythromycin, flurbiprofen, glipizide, glyburide, griseofulvin, ibuprofen, indinavir, indomethacin, itraconazole, ketoconazole, lansoprazole, lovastatin, mebendazole, naproxen, nelfinavir, ofloxacin, oxaprozin, phenazopyridine, phenytoin, piroxicam, raloxifene, ritonavir, saquinavir, sirolimus, spironolactone, tacrolimus, talinolol, tamoxifen, terfenadine, warfarin, amphotericin B, chlorthalidone, chlorothiazide, colistin, ciprofloxacin, furosemide, hydrochlorothiazide, mebendazole, methothrexate, neomycin, and enzalutamide.

In a preferred embodiment, the active ingredient is enzalutamide.

An organic solution, in this context, means that the respective solutes are dissolved in an organic phase, which requires the use of an organic solvent or solvent mixture. This does not per se exclude the presence of inorganic compounds. For example, an organic solution may comprise some water; however, the major part of the liquid phase is an organic solvent or solvent mixture. Preferably, the organic solution used in the method according to the present invention comprises not more than about 10 wt.-% water. In a further preferred embodiment, the organic solution comprises not more than about 5 wt.-% water, or not more than about 2 wt.-% of water, respectively. In a further preferred embodiment, the organic solution comprises no water or only residual amounts of water, such as not more than about 1 wt.-%. In this context, the weight percentages relate to the total weight of the organic solution.

An aqueous solution, in this context, means that the respective solutes are dissolved in water. In addition, an aqueous solution may optionally comprise a small amount of one or more organic solvents. Preferably, the aqueous solution comprises not more than about 5 wt.-% organic solvents, or not more than about 2 wt.-% of solvents, respectively. In a further preferred embodiment, the aqueous solution comprises no organic solvents or only residual amounts thereof, such as not more than about 1 wt.-%. In this context, the percentages are based on the total weight of the aqueous solution.

In a particularly preferred embodiment, the aqueous solution is a buffered aqueous solution. The expression "buffered" means that the aqueous solution comprises a buffer, such as an acid or an acidic salt in combination with a corresponding base or basic salt, wherein this combination increases the resistance of the buffered aqueous solution to a change of pH upon addition of (e.g. a further) acid or base. In principle, an active ingredient may also represent one of the components of a buffer system. More typically, however, a buffer system comprising two excipient components is used in the buffered aqueous solution used in the method according to the invention.

As mentioned, the buffered aqueous solution has a pH in the range from about 3 to about 9. Consequently, a buffer is used which is capable of maintaining such pH.

A further disclosure, which is not claimed, is directed to a method for producing nanoparticles, wherein the nanoparticles contain or consist of hydroxypropyl methylcellulose acetate succinate (HPMCAS), characterized in that i) HPMCAS is dissolved in an organic solvent in order to obtain an organic solution, ii) the organic solution is mixed, preferably diluted, with water to precipitate the nanoparticles in order to obtain precipitated nanoparticles and a mixed liquid phase.

As used herein, the mixing of the organic solution and the buffered aqueous solution, or water, includes active and/or passive mixing using a suitable mixing device, such as to allow the precipitation of the nanoparticles.

The mixing, which may in some cases also be understood as diluting, of the organic solution in ii) may be conducted quickly and/or intensively. Optionally, the mixing may involve stirring.

In a particularly preferred embodiment, the organic solution is provided in the form of a first fluid stream and the aqueous solution is provided in the form of a second fluid stream; and the mixing includes directing the first and the second fluid stream to contact one another. The fluid streams may, for example, be formed by conducting the respective fluids through channels or nozzles of a suitable fluidics or microfluidics device.

In a further preferred embodiment, the two fluid streams are ejected from nozzles, and the (ejected) fluid streams are directed such that the streams collide with, or impinge on one another. Such collision or impingement may be achieved by the appropriate orientation of the nozzles. For example, a MicroJet Reactor as described in EP 1 165 224 may be used.

In specific embodiments, in particular when the method is conducted on large scale, the organic solution is mixed with the buffered aqueous solution (or with water) as follows:

The organic solution and aqueous solution (or water) collide as impinging jets with a high velocity of more than 1 m/sec, where the Reynold number is higher than 500. The velocity, in one embodiment, may be higher than 50 m/sec as well. It is noted that the above indicated velocity is the velocity of each of the colliding streams, i.e., both the aqueous fluid stream and the fluid stream of the organic solution have this velocity.

The organic solution and the aqueous solution, or water, are preferably sprayed through nozzles usually smaller than about 1000 $\mu$m (for example smaller than about 500 $\mu$m or about 300 $\mu$m) with pressures of more than about 1 bar. Pressures of more than about 10 bars and even more than about 50 bar are suitable as well. The pressure may be regulated by pressure regulators.

The two streams collide in a reactor, where very rapid mixing takes place. Mixing times usually are below about 1 millisecond, preferably below about 0.5 milliseconds, or even below about 0.1 millisecond. The flow rates of the fluid streams may reach more than about 600 l/hour. Thus, the two impinging jets (or streams) collide in the reactor where precipitation takes place.

The mixing time is adjusted as a derivative of the flow rate: the higher the flow rates, the lower the mixing time will be. In the reactor, where the fluid streams collide, two liquid sheets plates are formed because of the parallel streams flowing against each other. After the collision, mixing occurs mainly through diffusion until the mixture is completed . This time period can be controlled with the flow rate and the gas pressure in the reactor. This kind of mixing is preferably obtained with a so called MicroJet reactor since its structure allows the collision of two streams in a free chamber under gas so that the particle size can be controlled.

The term "MicroJet reactor" includes all the geometries that are defined in WO 0061275 A2. The contents of this patent application is incorporated herein by reference. WO 0061275 A2 provides for a system for the initiation of chemical or physical processes including at least two liquid media to be injected by means of pumps, preferably high-pressure pumps, into a reactor chamber enclosed by a reactor housing and on to a shared collision point, each medium being injected through one nozzle. Through an opening in the reactor chamber, a gas, an evaporating liquid, a cooling liquid or a cooling gas, is introduced so as to maintain the gas atmosphere in the reactor interior, notably in the collision point of the liquid jets, and to cool the resulting products. The resulting products and excess gas are removed from the reactor housing via a further opening by positive pressure on the gas input side or negative pressure on the product and gas discharge side.

The organic solvent used in the method of the invention, e.g. for preparing an organic solution of the HPMCAS and the active ingredient, is selected taking into account, amongst other criteria, the specific grade of HPMCAS and the solubility profile of the active ingredient to be incorporated. The organic solvent may be (partly or fully) miscible with water. It is preferably selected from the group consisting of acetone, tetrahydrofuran, ethanol, methanol, and dichloromethane, or mixtures thereof. Examples of potentially suitable solvent mixtures include acetone/dichloromethane (e.g. 1:4 v/v), ethanol/dichloromethane (e.g. 1:1 v/v), and methanol/dichloromethane (e.g. 1:2 v/v). In further embodiments, the organic solvent is acetone, or comprises acetone.

In one specific embodiment, the active ingredient is enzalutamide and the organic solvent is acetone or a mixture of acetone and dichloromethane.

The solubility of three different commercially available HPMCAS grades in different solvents for use in the invention is depicted in Table 1 (from: Dow Chemicals, AFFINISOL™ HPMCAS for Spray-Dried Dispersion (SDD))

Table 1.

| Solvent | HPMCAS 716G | HPMCAS 912G | HPMCAS 126G |
|---|---|---|---|
| Deionized Water | Insoluble | Insoluble | Insoluble |
| Methanol | Soluble | Soluble | Soluble |
| Methanol/Dichloromethane (1:2) | Soluble | Soluble | Soluble |
| Ethanol | Insoluble | Insoluble | Partially |
| Ethanol/Water (4:1) | Soluble | Soluble | Soluble |
| Ethanol/Dichloromethane (1:1) | Soluble | Soluble | Soluble |
| Acetone | Soluble | Soluble | Soluble |
| Tetrahydrofuran | Soluble | Soluble | Soluble |
| Dichloromethane | Soluble | Soluble | Soluble |

As mentioned, the organic solution is a solution comprising the organic solvent, HPMCAS, and preferably at least one active ingredient. The concentration of the active ingredient is preferably relatively high in relation to the saturation concentration of the active ingredient in the respective organic solution - i.e. also taking any other constituents of the organic solutions (e.g. including the HPMCAS) into account. Such saturation concentration in an otherwise comparable organic phase may also be referred to as the corresponding saturation concentration. In a preferred embodiment, the concentration of the active ingredient in the organic solution is at least about 10% of its corresponding saturation concentration at normal temperature and pressure. Also preferred are embodiments in which the concentration of the active ingredient in the organic solution is at least about 20% of its corresponding saturation concentration, or even at least about 30%, respectively, at normal temperature and pressure.

As used herein, the normal temperature and pressure mean are 20°C and 760 mm Hg, respectively, as defined by the National Institute of Standards and Technology (NIST). In further embodiments, the concentration of the active ingredient is in the range of about 30% to about 99% of the corresponding saturation concentration, or in the range from about 50% to about 95% of the corresponding saturation concentration, respectively.

According to another preferred embodiment, the concentration of the active ingredient in the organic solution is not higher than the concentration of the HPMCAS in the organic solution, wherein the concentrations are expressed as weight per volume. In other words, the weight ratio of the HPMCAS to the active ingredient in the organic solution is at least about 1 in this specific embodiment. In other embodiments, the weight ratio of the HPMCAS to the active ingredient is in the range from about 1 to about 5, or it is about 2, about 3, or about 4, respectively.

In another preferred embodiment, the concentration of HPMCAS in the organic solution is 1 to 100 mg/mL, preferably 5 to 30 mg/mL, and optionally the concentration of the at least one active ingredient in the organic solution is 1 to 30 mg/mL, preferably 2 to 15 mg/mL (per active ingredient). The inventors have found that these concentrations provide good results.

The mixed liquid phase preferably comprises water and the organic solvent.

In the context of the present invention, the term "water" comprises water, pure water, deionized water, and to some extent aqueous solutions. Aqueous solutions are in particular solutions of inorganic salts or water-soluble organic compounds and/or surfactants, in particular of acids such as carboxylic acids and/or their conjugated bases such as acetic acid and acetate. Aqueous solutions containing organic solvents in less than 6.1 or 10 wt.-% may also be referred to as "water".

In other words, a buffered aqueous solution may also be referred to as "water". As mentioned, buffers typically include an acid or acidic salt in combination with a base or basic salt. It is clear to a skilled person that potentially suitable buffers should be selected in view of the pH which an aqueous solution should be adjusted to. For example, an acetate (e.g. sodium acetate) buffer comprising acetic acid and sodium acetate is useful in the range from about pH 3.6 to about 5.8. For adjusting to pH values in the range of approx. 6 to 8, certain types of phosphate buffer and citrate buffer may be useful. In one of the preferred embodiments, the buffered aqueous solution comprises acetate buffer.

In one specific embodiment, the active ingredient is enzalutamide, the organic solvent is acetone or a mixture of acetone and dichloromethane, and the aqueous solution is buffered with acetate buffer.

In a preferred embodiment, the aqueous solution, or the water, comprises a carboxylic acid and/or conjugated base, preferably acetic acid and/or acetate. Preferably, the total concentration of carboxylic acid and conjugated base, preferably acetic acid and acetate, is 5 to 1000 mM, more preferably 20 to 250 mM or 20 to 500 mM. Surprisingly, the inventors have found that the addition of a carboxylic acid and/or conjugated base to the water can substantially facilitate the formation of a nanoparticle precipitate and avoids formation of one plastic mass from which no nanoparticles can be obtained.

As mentioned, the buffered aqueous solution has a pH in the range from about 3 to about 9. In a preferred embodiment, the pH value of the aqueous solution, or of the water, is between 5 and 8, or between 6 and 7.

In another preferred embodiment, the pH value of the water is between 5 and 8, preferably between 6 and 7. This is in particular preferred, if no active ingredient is dissolved in the organic solvent.

In a further preferred embodiment, the pH value of the water is between 6 and 8, preferably between 6 and 7. This is in particular preferred, if at least one active ingredient is dissolved in the organic solvent prior to ii).

The solubility of HPMCAS increases as the pH increases. Solubility of HPMCAS is notable from pH 6 and higher. Therefore, the person skilled in the art would choose a low pH value, in particular below 5. However, the inventors have surprisingly found that good results can be obtained at the indicated pH values between pH 5 and 8, or in the range from pH 5 to pH 7.

The pH values given in the preceding paragraph in particular refer to HPMCAS with a degree of substitution at the cellulose backbone (ii) between 5 and 15 %, preferably 7 and 11 %, with acetate, and between 8 and 18 %, preferably 10 and 14 %, with succinate (e.g. HPMCAS 912G). When reference is made in the preceding paragraph to an active ingredient, the active ingredient is preferably enzalutamide.

In a preferred embodiment, the degree of substitution at the cellulose backbone of the HPMCAS is (ii) between 5 and 15 %, preferably 7 and 11 %, with acetate, and between 8 and 18 %, preferably 10 and 14 %, with succinate (e.g. HPMCAS 912G).

It is pointed out that the given pH values may be rounded up or down, i.e. the range of the pH value between 6 and 7 comprises pH values between 5.5 and 7.49.

In one specific embodiment, the method of the invention is performed with the active ingredient being enzalutamide, the organic solvent being acetone or a mixture of acetone and dichloromethane, and the buffered aqueous solution having a pH in the range from about 5 to 7. Optionally, the organic solution and/or the aqueous solution may comprise further solutes, in particular compounds useful for further improving the method of the invention or excipients useful for improving the stability or performance of the nanoparticles. For example, the incorporation of a surfactant may be useful.

In a further preferred embodiment, therefore, the organic solution and/or the aqueous solution, or the water, contains at least one surfactant. The at least one surfactant can potentially stabilize a HPMCAS particle by adsorption on its surface.

Potentially suitable surfactants include pharmaceutically acceptable nonionic surfactants, such as polysorbates or poloxamers, and pharmaceutically acceptable ionic surfactants, such as phospholipids or sodium dodecyl sulfate.

In a preferred embodiment, the ratio by volume of the organic solution to that of the aqueous phase in step c) (or water in step ii) is from 1 to between 2 and 10, or from 1 to between 3 and 7. The inventors have found that these ratios provide good results.

In other preferred embodiments, the ratio of the volume of the buffered aqueous solution to the volume of the organic solution in step c) is at least 1, and preferably from [2 to 20?] or from [3 to 10?], respectively.

In a further preferred embodiment, the ratio of the volume of the buffered aqueous solution to the volume of the organic solution in step c) is selected such as to lead to a mixed liquid phase upon mixing. This mixed liquid phase comprises the water that was comprised in the aqueous solution and the organic solvent that was comprised in the organic solution before mixing. In order to form a mixed liquid phase, the liquid constituents of the organic solution and the aqueous solution must be miscible at the selected volume ratio.

Alternatively, the ratio of the volume of the buffered aqueous solution to the volume of the organic solution in step c) may be selected such that, upon mixing, a system comprising two liquid phases, e.g. an emulsion, is generated.

In either of these embodiments, the precipitated nanoparticles may form a solid phase which is dispersed within the mixed liquid phase or within the system comprising two liquid phases. The nanoparticles formed as described above may then be further processed to the final pharmaceutical composition. In case that the final pharmaceutical composition is tablet, capsule or powder for oral application, this can be realized by using a spray drying or granulation process for solidification of the suspension of nanoparticles.

Therefore, in some of the preferred embodiments, the method of the invention further comprises a step of d) isolating or drying the nanoparticles. Any suitable isolation method may be used, such as filtration (e.g. tangential flow filtration), or any suitable drying method, such as spray drying or freeze drying.

In a preferred embodiment, which is not claimed, the method is a method for producing nanoparticles for oral application in a human being. In a preferred embodiment, the method comprises iii) spray drying or granulating the precipitated nanoparticles and the mixed liquid phase and optionally preparing a tablet, capsule, or powder for oral administration. In a preferred embodiment, the method comprises iii) spray drying or granulating the precipitated nanoparticles and the mixed liquid phase to obtain a dry product and optionally preparing a tablet, capsule, or powder for oral administration from the dry product. Not all nanoparticles are suitable for oral application. In contrast, the present method provides particles, which are particularly well suited for oral application, since the small particle size and large surface area allows fast and complete dissolution of active ingredients with low solubility in the gastrointestinal tract, especially in the intestine.

In a preferred embodiment, at least one active ingredient is dissolved in the organic solvent respectively organic solution prior to ii), wherein the precipitated nanoparticles contain the at least one active ingredient, and wherein the nanoparticles contain or consist of HPMCAS and the at least one active ingredient. Preferably, the at least one active ingredient is one active ingredient.

The present invention allows embedding active ingredients in the HPMCAS matrix, which has a large surface area.

In a further aspect, which is not claimed, the present disclosure is directed to nanoparticles obtainable by the method of the invention. In particular, the nanoparticles are useful for oral administration to a human subject.

The nanoparticles obtainable according to the invention preferably comprise a solid dispersion of the active ingredient in the HPMCAS. As used herein, a solid dispersion means that a matrix of a solid material (in this case the HPMCAS) incorporates a homogeneously dispersed material (in this case the active ingredient) in dissolved or colloidal form. A solid dispersion may in principle exist as a large unit, such as an extrudate, or in the form of microparticles. According to the invention, however, it exists in the form of nanoparticles, which are obtainable by the method disclosed herein. This brings about a substantial advantage for the oral delivery of poorly soluble active ingredients in that synergies are achieved between the benefits of nanoparticles, which generally exhibit rapid drug dissolution based on their extremely large specific surface area (even compared to microparticles), and the benefits of solid dispersions, which achieve enhanced drug dissolution due to the fact that they contain an active ingredient in its dissolved (or colloidally dissolved) form.

In a further preferred embodiment, the nanoparticles obtainable by the method of the invention are amorphous. The amorphous structure can be determined by Differential Scanning calorimetry (DSC) or X-ray diffraction analysis. In particular, the active ingredient comprised in the nanoparticles is at least partially amorphous. Preferably, the active ingredient exhibits a low degree of crystallinity. In one of the preferred embodiments, the active ingredient comprised in the nanoparticles is predominantly amorphous, which means that the degree of crystallinity of the active ingredient in the nanoparticles is less than about 50%. In another preferred embodiment, the degree of crystallinity of the active ingredient is not more than about 30%, or not more than about 20%, or not more than about 10%, respectively. Further preferred is an embodiment in which the active ingredient is substantially amorphous.

In one embodiment, the nanoparticles obtainable by the method according to the invention consist of HPMCAS and the active ingredient. In an alternative embodiment, the nanoparticles comprise one or more further inactive ingredients, such as a surfactant.

The nanoparticles obtainable according to the invention are in particular nanoparticles suitable for oral application to a human being, further characterized in that the nanoparticles have a particle size (expressed as z-average) of 100 to 900 nm, preferably of 150 to 750 nm, or of 200 to 600 nm, measured by Dynamic Light Scattering. Also preferred are particle sizes expressed as z-averages in the range from about 100 nm to about 600 nm, or from about 150 nm to about 500 nm, respectively. The specific particle size can be obtained by the method of the present invention. As mentioned, the specific surface area (i.e. weight-specific surface area) of such particles is high, which improves the bioavailability and dissolution rate and enables immediate release in the gastrointestinal tract, more preferably the intestine, of poorly water-soluble active ingredients.

In a preferred embodiment, the nanoparticles of the invention or obtainable by the method of the invention have a polydispersity index of below 0.8, such as 0.1 to 0.8, preferably of 0.15 to 0.7 or 0.6, measured by Dynamic Light Scattering. In other preferred embodiments, the polydispersity index of the nanoparticles is the range from about 0.1 to about 0.5, or from about 0.1 to about 0.3, respectively. The specific polydispersity index can be obtained by the method of the present invention.

In a preferred embodiment, the nanoparticles obtainable by the method of the invention have a spongy structure. The method of the present invention results in a specific HPMCAS polymer matrix with a sponge-like scaffold with a large surface area. This structure can be determined e.g. by x-ray microscopy.

In a preferred embodiment, the nanoparticles contain at least one active ingredient or consist of HPMCAS and at least one active ingredient. In a specific preferred embodiment, the nanoparticles contain one active ingredient or consist of HPMCAS and one active ingredient. In a specific preferred embodiment, the nanoparticles contain one or more excipients, such as surfactants.

In a further aspect, which is not claimed, the present disclosure is directed to a pharmaceutical composition comprising the nanoparticles of the invention or obtainable by the method of the invention.

In a preferred embodiment, the pharmaceutical composition is a composition for oral application. In particular, it is formulated and processed such as to be suitable for oral administration. Furthermore, it may be used in the manufacture of a medicament for oral administration.

In a preferred embodiment, the pharmaceutical composition is a composition for application in a human being, tablet, capsule, or powder for oral administration. In a preferred embodiment, the pharmaceutical composition is a tablet, capsule, powder, or suspension, preferably an aqueous suspension, more preferably a tablet, capsule, or powder. The advantage of the invention is that it can be readily administered and the absorption of the active ingredient may begin immediately in the gastrointestinal tract, more preferably the intestine.

In a preferred embodiment, release of the active ingredient takes place in the gastrointestinal tract, more preferably the intestine.

In a further aspect, which is not claimed, the present disclosure is directed to the use of the nanoparticles of the invention or obtainable by the method of the invention for

    a) a pharmaceutical composition with immediate release in the gastrointestinal tract, more preferably the intestine, and/or
    b) the oral application of an active ingredient, preferably in a human being, and/or
    c) increasing bioavailability of an active ingredient in a pharmaceutical composition for oral application and/or
    d) optionally coupling to an active ingredient.

Within this disclosure, the verbs "to comprise" and "to contain" comprise the verb "to consist of" and its conjugations. In other words, the terms "comprise", "comprises", "comprising", "contain", "contains", "containing" etc. are open expressions used as synonyms. They cover the meaning of closed expressions such as "consisting of", even though their scope is not restricted to such closed terms.

The invention is directed to one nanoparticle or several nanoparticles.

Further, preferred embodiments are defined in the claims.

Examples

Examples 1 to 8. (reference)

These Examples were conducted in line with these method steps:

i) HPMCAS (AFFINISOL HPMCAS 912G from Dow chemicals) was dissolved in acetone in order to obtain an organic solution and

ii) the organic solution was quickly mixed under intensive stirring with water to precipitate the nanoparticles in order to obtain precipitated nanoparticles and a mixed liquid phase. The concentration of HPMCAS in the organic solution was 15 mg/mL. The ratio by volume of the organic solution to water was 1 to 5.

In Examples 1 to 5, no active ingredient was dissolved in the acetone respectively organic solution.

In Examples 6 to 8, the active ingredient enzalutamide was dissolved in the acetone respectively organic solution prior to ii). The concentration of the active ingredient in the organic solution was 5 mg/mL.

In Examples 2 to 8, the water comprised 50 mM of acetic acid and/or acetate (50 mM of total carboxylic acid plus conjugated base).

Further parameters, such as the pH value of the water, the z-average particle size, the polydispersity index is depicted in Table 2:

Table 2.

| Example | Active Ingredient | Acetic acid and/or acetate in water (mM) | pH | Particle size (nm) | PDI |
|---|---|---|---|---|---|
| 1 | No | - | 7 | - | - |
| | | | | | |
| 2 | No | 50 | 4 | - | - |
| 3 | No | 50 | 5 | 209 | 0.189 |
| 4 | No | 50 | 6 | 384 | 0.308 |
| 5 | No | 50 | 7 | 347 | 0.384 |
| | | | | | |
| 6 | Yes | 50 | 5 | - | - |
| 7 | Yes | 50 | 6 | 423 | 0.244 |
| 8 | Yes | 50 | 7 | 582 | 0.557 |

In Examples 1, 2, and 6, formation of one plastic mass was observed. No nanoparticles or no precipitate of nanoparticles could be obtained.

In Examples 3 to 5 and 7 to 8, nanoparticles were precipitated. The nanoparticles were amorphous and had a spongy structure.

In Examples 3 to 5, the nanoparticles consist of HPMCAS only.

In Examples 6 to 8, the nanoparticles consist of HPMCAS and the active ingredient.

Examples 9 to 13

In this series of examples, a MicroJet Reactor (MJR®) was used to prepare nanoparticles comprising HMPCAS (Aqoat AS-MG, Shin-Etsu Chemical) and either itraconazole (ITZ), aprepitant (APT), or furosemide (FRS) as active ingredient.

For each experiment, the organic solution was prepared by dissolving the respective active ingredient (5 mg/mL) and the HPMCAS (15 mg/mL) in acetone such as to yield the specified concentrations and a weight ratio of active ingredient to HPMCAS of 3. The aqueous solution consisted of aqueous acetate buffer adjusted to pH 5.

The organic solutions and the buffered aqueous solutions were provided as fluid streams and mixed by impingement using a MicroJet Reactor having nozzles with pinhole diameters of 100 $\mu$m for the organic solution and 200 $\mu$m for the aqueous solution, respectively. The maximum pressure of the fluid streams were set to either 7 bar or 14 bar, and the volume ratio of the aqueous solution to that of the organic solution was set to either 4 or 5.

Subsequently, the nanoparticle suspensions obtained upon mixing were characterized with respect to their particle sizes (expressed as z-average) and polydispersity indices (PDI) using a Malvern Zetasizer. For the particle size measurements, the nanoparticle suspensions were diluted 1 : 10 with acetate buffer solution pH 5. For examples 11 to 13, these measurements were repeated after 24 h of storage at 5 °C in order to evaluate the stability of the nanoparticles.

The results are shown in Table 3:

Table 3

| Ex. | API | Ratio aqueous to organic phase | Max. pressure [bar] | z-average [nm] | PDI | z-average after 24 h [nm] | PDI after 24 h |
|---|---|---|---|---|---|---|---|
| 9 | ITZ | 4 | Z | 288.4 | 0.111 | n/d | n/d |
| 10 | ITZ | 4 | 14 | 516.5 | 0.111 | n/d | n/d |
| 11 | ITZ | 5 | 7 | 193.8 | 0.114 | 192.0 | 0.097 |
| 12 | APT | 5 | 7 | 198.3 | 0.254 | 556.7 | 0.438 |
| 13 | FRS | 5 | 7 | 207.0 | 0.252 | 244.9 | 0.238 |

**Claims**

1.  A method for producing nanoparticles comprising an active ingredient and hydroxypropyl methylcellulose acetate succinate (HPMCAS), the method comprising the steps of:

    a) providing an organic solution of the active ingredient and the HPMCAS in an organic solvent;
    b) providing an aqueous solution having a pH of 3 to 9;
    c) mixing the organic solution with the aqueous solution such as to precipitate the nanoparticles; wherein

      - the organic solution is provided in the form of a first fluid stream;
      - the aqueous solution is provided in the form of a second fluid stream;

    and wherein the mixing includes directing the first and the second fluid stream to contact one another; and wherein each of the first and the second fluid stream is ejected from a nozzle, and wherein the fluid streams are directed such as to impinge on one another;
    and optionally, wherein the method comprises a further step of
    d) isolating and/or drying the nanoparticles.

2.  The method according to claim 1, wherein the aqueous solution is a buffered aqueous solution; optionally wherein the buffered aqueous solution comprises acetate buffer.

3.  The method according to claim 1 or 2, wherein the mixing is performed in a fluidics or microfluidics device; or wherein the mixing is performed in a reactor providing for the collision of the two fluid streams in a free chamber under gas; optionally wherein said reactor is a system comprising a reactor chamber enclosed by a reactor housing, a pumping means allowing for at least two liquid media to be injected on to a shared collision point into the reactor chamber, preferably high-pressure pumps, each medium being ejected through one nozzle; further optionally wherein the reactor chamber has an opening for the introduction of a gas, evaporating liquid, cooling liquid, or a cooling gas to the reactor interior, and wherein the reactor housing has a further opening for removal of resulting products and excess gas either by positive pressure on the gas input or negative pressure on the product and gas discharge.

4.  The method according to any one of the preceding claims, wherein the nanoparticles comprise of a solid dispersion of the active ingredient in the HPMCAS; and/or wherein the active ingredient comprised in the nanoparticles is at least partially amorphous; and/or wherein the nanoparticles essentially consist of the active ingredient and the HPMCAS.

5.  The method according to any one of the preceding claims, wherein the nanoparticles have a particle size expressed as z-average of 100 to 900 nm, or 150 to 750 nm, or from 100 to 600 nm, as measured by dynamic light scattering; or wherein the nanoparticles have a polydispersity index of 0.1 to 0.8, or of 0.1 to 0.5, as measured by dynamic light scattering.

6.  The method according to any one of the preceding claims, wherein the active ingredient:

    - is not more soluble in water than sparingly soluble, and is preferably slightly soluble, very slightly soluble, practically insoluble, wherein said terms are defined by the volume (ml) of water needed to dissolve 1 g of active ingredient, which is 30 to 100 for "sparingly soluble", 100 to 1000 for "slightly soluble", 1000 to 10000 for "very slightly soluble", and > 10000 for "practically insoluble"; or
- is a class 2 or class 4 compound according to the Biopharmaceutical Classification System (BCS) or to the Biopharmaceutical Drug Disposition Classification System (BDDCS); or wherein
- the active ingredient is selected from amiodarone, atorvastatin, azithromycin, carbamazepine, carvedilol, chlorpromazine, cisapride, ciprofloxacin, cyclosporine, danazol, dapsone, diclofenac, diflunisal, digoxin, erythromycin, flurbiprofen, glipizide, glyburide, griseofulvin, ibuprofen, indinavir, indomethacin, itraconazole, ketoconazole, lansoprazole, lovastatin, mebendazole, naproxen, nelfinavir, ofloxacin, oxaprozin, phenazopyridine, phenytoin, piroxicam, raloxifene, ritonavir, saquinavir, sirolimus, spironolactone, tacrolimus, talinolol, tamoxifen, terfenadine, warfarin, amphotericin B, chlorthalidone, chlorothiazide, colistin, ciprofloxacin, furosemide, hydrochlorothiazide, mebendazole, methothrexate, neomycin, and enzalutamide.

7. The method according to any one of the preceding claims, wherein the concentration of the active ingredient in the organic solution is at least 20% of its corresponding saturation concentration at normal temperature and pressure.

8. The method according to any one of the preceding claims, wherein the concentration of the active ingredient in the organic solution is not higher than the concentration of the HPMCAS in the organic solution, wherein the concentrations are expressed as weight per volume.

9. The method according to any one of the preceding claims, wherein the organic solvent is selected from acetone, tetrahydrofuran, ethanol, methanol, dichloromethane, and mixtures thereof.

10. The method according to any one of the preceding claims, wherein the buffered aqueous solution has a pH of 5 to 8, or 5 to 7.

11. The method according to any one of the preceding claims, wherein the buffered aqueous solution and/or the organic solution further comprises a surfactant; optionally wherein the surfactant is selected from pharmaceutically acceptable ionic or nonionic surfactants, wherein the ionic surfactants include, without limitation, phospholipids and sodium dodecyl sulfate, and wherein the nonionic surfactants include, without limitation, polysorbates and poloxamers.

12. The method according to any one of the preceding claims, wherein in step c) the ratio of the volume of the buffered aqueous solution to the volume of the organic solution is at least 1, and preferably from 2 to 20, or from 3 to 10, respectively.

13. The method according to any one of the preceding claims, wherein the organic solution and aqueous solution collide as impinging jets with a high velocity of more than 1 m/sec, where the Reynold number is higher than 500; or wherein the velocity is higher than 50 m/sec, wherein said velocity is the velocity of each of the colliding streams.

14. The method according to any one of the preceding claims, wherein the organic solution and the aqueous solution are sprayed through nozzles smaller than 1000 $\mu$m, or smaller than 500 $\mu$m, or smaller than 300 $\mu$m, at pressures of more than about 1 bar, or optionally more than about 10 bar, or more than about 50 bar.

15. The method according to any one of the preceding claims, wherein the mixing times of the two streams is below about 1 millisecond, preferably below about 0.5 milliseconds, or below about 0.1 millisecond.

**Patentansprüche**

1. Verfahren zum Herstellen von Nanopartikeln, die einen Wirkstoff und Hydroxypropylmethylcellulosacetatsuccinat (HPMCAS) umfassen, wobei das Verfahren die folgenden Schritte umfasst:

a) Bereitstellen einer organischen Lösung des Wirkstoffs und des HPMCAS in einem organischen Lösungsmittel;
b) Bereitstellen einer wässrigen Lösung mit einem pH-Wert von 3 bis 9;
c) Mischen der organischen Lösung mit der wässrigen Lösung, um die Nanopartikel auszufällen; wobei

- die organische Lösung in Form eines ersten Fluidstroms bereitgestellt ist;
- die wässrige Lösung in Form eines zweiten Fluidstroms bereitgestellt ist;
und wobei das Mischen das Leiten des ersten und des zweiten Fluidstroms derart, dass sie in Kontakt miteinander kommen, umfasst; und wobei jeder von dem ersten und dem zweiten Fluidstrom aus einer Düse ausgestoßen wird und wobei die Fluidströme derart geleitet werden, dass sie aufeinander auftreffen; und wobei das Verfahren gegebenenfalls einen weiteren Schritt des

d) Isolierens und/oder Trocknens der Nanopartikel umfasst.

2. Verfahren nach Anspruch 1, wobei die wässrige Lösung eine gepufferte wässrige Lösung ist; wobei die gepufferte wässrige Lösung gegebenenfalls Acetatpuffer umfasst.

3. Verfahren nach Anspruch 1 oder 2, wobei das Mischen in einer Fluidik- oder Mikrofluidik-Vorrichtung durchgeführt wird; oder wobei das Mischen in einem Reaktor durchgeführt wird, der die Kollision der zwei Fluidströme in einer freien Kammer unter Gas bereitstellt; wobei der Reaktor gegebenenfalls ein System ist, das eine Reaktorkammer, die von einem Reaktorgehäuse eingeschlossen ist, ein Pumpmittel, das ermöglicht, dass mindestens zwei flüssige Medien an einem gemeinsamen Kollisionspunkt in die Reaktorkammer eingespritzt werden, vorzugsweise Hochdruckpumpen, wobei jedes Medium durch eine Düse ausgestoßen wird, umfasst; wobei die Reaktorkammer ferner gegebenenfalls eine Öffnung zur Einbringung eines Gases, einer Verdampfungsflüssigkeit, Kühlflüssigkeit oder eines Kühlgases zum Reaktorinneren umfasst und wobei das Reaktorgehäuse eine weitere Öffnung zur Entfernung von entstandenen Produkten und überschüssigem Gas entweder durch Überdruck am Gaseinlass oder Unterdruck am Produkt- und Gasauslass umfasst.

4. Verfahren nach einem der vorstehenden Ansprüche, wobei die Nanopartikel eine feste Dispersion des Wirkstoffs in dem HPMCAS umfassen; und/oder wobei der Wirkstoff, den die Nanopartikel umfassen, zumindest teilweise amorph ist; und/oder wobei die Nanopartikel im Wesentlichen aus dem Wirkstoff und dem HPMCAS bestehen.

5. Verfahren nach einem der vorstehenden Ansprüche, wobei die Nanopartikel eine als Z-Durchmesser ausgedrückte Partikelgröße von 100 bis 900 nm, oder 150 bis 750 nm, oder von 100 bis 600 nm bei Messung mittels dynamischer Lichtstreuung aufweisen; oder wobei die Nanopartikel einen Polydispersitätsindex von 0,1 bis 0,8 oder von 0,1 bis 0,5 bei Messung mittels dynamischer Lichtstreuung aufweisen.

6. Verfahren nach einem der vorstehenden Ansprüche, wobei der Wirkstoff:

- in Wasser nicht stärker löslich als schwer löslich ist und vorzugsweise geringfügig löslich, sehr geringfügig löslich, praktisch unlöslich ist, wobei die Ausdrücke durch das Volumen (ml) an Wasser definiert werden, das zum Auflösen von 1 g Wirkstoff benötigt wird, was 30 bis 100 für "schwer löslich", 100 bis 1000 für "geringfügig löslich", 1000 bis 10000 für "sehr geringfügig löslich" und > 10000 für "praktisch unlöslich" ist; oder
- eine Verbindung der Klasse 2 oder Klasse 4 gemäß dem Biopharmaceutical Classification System (BCS) oder dem Biopharmaceutical Drug Disposition Classification System (BDDCS); oder wobei
- der Wirkstoff aus Amiodaron, Atorvastatin, Azithromycin, Carbamazepin, Carvedilol, Chlorpromazin, Cisaprid, Ciprofloxacin, Cyclosporin, Danazol, Dapson, Diclofenac, Diflunisal, Digoxin, Erythromycin, Flurbiprofen, Glipizid, Glyburid, Griseofulvin, Ibuprofen, Indinavir, Indomethacin, Itraconazol, Ketoconazol, Lansoprazol, Lovastatin, Mebendazol, Naproxen, Nelfinavir, Ofloxacin, Oxaprozin, Phenazopyridin, Phenytoin, Piroxicam, Raloxifen, Ritonavir, Saquinavir, Sirolimus, Spironolacton, Tacrolimus, Talinolol, Tamoxifen, Terfenadin, Warfarin, Amphotericin B, Chlorthalidon, Chlorthiazid, Colistin, Ciprofloxacin, Furosemid, Hydrochlorothiazid, Mebendazol, Methothrexat, Neomycin und Enzalutamid ausgewählt ist.

7. Verfahren nach einem der vorstehenden Ansprüche, wobei die Konzentration des Wirkstoffs in der organischen Lösung bei mindestens 20 % seiner entsprechenden Sättigungskonzentration bei Normaltemperatur und -druck liegt.

8. Verfahren nach einem der vorstehenden Ansprüche, wobei die Konzentration des Wirkstoffs in der organischen Lösung nicht höher als die Konzentration des HPMCAS in der organischen Lösung ist, wobei die Konzentrationen als Gewicht pro Volumen angegeben sind.

9. Verfahren nach einem der vorstehenden Ansprüche, wobei das organische Lösungsmittel ausgewählt ist aus Aceton, Tetrahydrofuran, Ethanol, Methanol, Dichlormethan und Gemischen davon.

10. Verfahren nach einem der vorstehenden Ansprüche, wobei die gepufferte wässrige Lösung einen pH-Wert von 5 bis 8, oder 5 bis 7, aufweist.

**11.** Verfahren nach einem der vorstehenden Ansprüche, wobei die gepufferte wässrige Lösung und/oder die organische Lösung ferner ein Tensid umfasst; wobei das Tensid gegebenenfalls ausgewählt ist aus pharmazeutisch annehmbaren ionischen oder nichtionischen Tensiden, wobei zu den ionischen Tensiden, ohne dass dies jedoch eine Einschränkung darstellt, Phospholipide und Natriumdodecylsulfat zählen und wobei zu den nichtionischen Tensiden, ohne dass dies jedoch eine Einschränkung darstellt, Polysorbate und Poloxamere zählen.

**12.** Verfahren nach einem der vorstehenden Ansprüche, wobei in Schritt c) das Verhältnis des Volumens der gepufferten wässrigen Lösung zum Volumen der organischen Lösung bei mindestens 1, und vorzugsweise 2 bis 20 bzw. 3 bis 10, liegt.

**13.** Verfahren nach einem der vorstehenden Ansprüche, wobei die organische Lösung und die wässrige Lösung als aufeinander auftreffende Strahle mit einer hohen Geschwindigkeit von 1 m/s aufeinander auftreffen, wobei die Reynolds-Zahl höher als 500 ist; oder wobei die Geschwindigkeit höher als 50 m/s ist, wobei die Geschwindigkeit die Geschwindigkeit jedes der kollidierenden Ströme ist.

**14.** Verfahren nach einem der vorstehenden Ansprüche, wobei die organische Lösung und die wässrige Lösung durch Düsen, die kleiner als 1000 $\mu$m, oder kleiner als 500 $\mu$m oder kleiner als 300 $\mu$m, sind, bei Drücken von mehr als etwa 1 bar, oder gegebenenfalls mehr als etwa 10 bar oder mehr als etwa 50 bar, gesprüht werden.

**15.** Verfahren nach einem der vorstehenden Ansprüche, wobei die Mischzeiten der zwei Ströme unter etwa 1 Millisekunde, vorzugsweise unter etwa 0,5 Millisekunden oder unter etwa 0,1 Millisekunde, liegen.

**Revendications**

**1.** Procédé de production de nanoparticules comprenant un principe actif et de l'acétate succinate d'hydroxypropylméthylcellulose (HPMCAS), le procédé comprenant les étapes suivantes :

    a) fournir une solution organique du principe actif et du HPMCAS dans un solvant organique ;
    b) fournir une solution aqueuse ayant un pH de 3 à 9 ;
    c) mélanger la solution organique à la solution aqueuse de manière à précipiter les nanoparticules ;

        - la solution organique étant fournie sous la forme d'un premier flux de fluide ;
        - la solution aqueuse étant fournie sous la forme d'un deuxième flux de fluide ;

    et le mélange incluant une étape visant à diriger le premier et le deuxième flux de fluide pour qu'ils entrent en contact l'un avec l'autre ; et chacun des premier et deuxième flux de fluide étant éjecté d'une buse, et les flux de fluide étant dirigés de manière à entrer en collision l'un avec l'autre ;
    et facultativement, le procédé comprenant une étape supplémentaire visant à
    d) isoler et/ou sécher les nanoparticules.

**2.** Procédé selon la revendication 1, la solution aqueuse étant une solution aqueuse tamponnée ; facultativement, la solution aqueuse tamponnée comprenant un tampon acétate.

**3.** Procédé selon la revendication 1 ou 2, le mélange étant effectué dans un dispositif fluidique ou micro-fluidique ; ou le mélange étant effectué dans un réacteur assurant la collision des deux flux de fluide dans une chambre libre sous atmosphère gazeuse ; facultativement, ledit réacteur étant un système comprenant une chambre de réacteur enfermée par un boîtier de réacteur, des moyens de pompage permettant à au moins deux milieux liquides d'être injectés en un point de collision partagé dans la chambre de réacteur, de préférence des pompes à haute pression, chaque milieu étant éjecté par une buse ; en outre, facultativement, la chambre de réacteur comportant une ouverture destiné à l'introduction d'un gaz, d'un liquide d'évaporation, d'un liquide de refroidissement ou d'un gaz de refroidissement à l'intérieur du réacteur, et le boîtier du réacteur comportant une autre ouverture destinée à l'élimination des produits résultants et du gaz en excès soit par pression positive sur l'entrée de gaz, soit par pression négative sur l'évacuation du produit et du gaz.

**4.** Procédé selon l'une quelconque des revendications précédentes, les nanoparticules comprenant une dispersion solide du principe actif dans le HPMCAS ; et/ou le principe actif compris dans les nanoparticules étant au moins partiellement amorphe; et/ou les nanoparticules comprenant essentiellement le principe actif et le HPMCAS.

**5.** Procédé selon l'une quelconque des revendications précédentes, les nanoparticules ayant une taille de particule exprimée en z-moyenne de 100 à 900 nm, ou de 150 à 750 nm, ou de 100 à 600 nm, telle que mesurée par diffusion de lumière dynamique ; ou les nanoparticules ayant un indice de polydispersité de 0,1 à 0,8, ou de 0,1 à 0,5, tel que mesuré par diffusion de lumière dynamique.

**6.** Procédé selon l'une quelconque des revendications précédentes, le principe actif :

- n'étant pas plus soluble dans l'eau que modérément soluble, et étant de préférence légèrement soluble, très légèrement soluble, pratiquement insoluble, lesdits termes étant définis par le volume (ml) d'eau nécessaire pour dissoudre 1 g de principe actif, qui est de 30 à 100 pour « peu soluble », 100 à 1000 pour « légèrement soluble », 1000 à 10000 pour « très légèrement soluble » et > 10000 pour « pratiquement insoluble »; ou
- étant un composé de classe 2 ou de classe 4 selon le système de classification biopharmaceutique (BCS) ou le système de classification de disposition des médicaments biopharmaceutiques (BDDCS) ; ou
- le principe actif étant choisi parmi l'amiodarone, l'atorvastatine, l'azithromycine, la carbamazépine, le carvédilol, la chlorpromazine, le cisapride, la ciprofloxacine, la cyclosporine, le danazol, la dapsone, le diclofénac, le diflunisal, la digoxine, l'érythromycine, le flurbiprofène, le glipizide, le glyburide, la griséofulvine, l'ibuprofène, l'indinavir, l'indométacine, l'itraconazole, le kétoconazole, le lansoprazole, la lovastatine, le mébendazole, le naproxène, le nelfinavir, l'ofloxacine, la oxaprozine, la phénazopyridine, la phénytoïne, le piroxicam, le raloxifène, le ritonavir, le saquinavir, le sirolimus, le spironolactone, le tacrolimus, le talinolol, le tamoxifène, la terfénadine, la warfarine, l'amphotéricine B, le chlorthalidone, le chlorothiazide, la colistine, la ciprofloxacine, le furosémide, l'hydrochlorothiazide, le mébendazole, le méthothrexate, la néomycine et l'enzalutamide.

**7.** Procédé selon l'une quelconque des revendications précédentes, la concentration du principe actif dans la solution organique étant d'au moins 20 % de sa concentration de saturation correspondante à température et pression normales.

**8.** Procédé selon l'une quelconque des revendications précédentes, la concentration du principe actif dans la solution organique n'étant pas supérieure à la concentration du HPMCAS dans la solution organique, les concentrations étant exprimées en poids par volume.

**9.** Procédé selon l'une quelconque des revendications précédentes, le solvant organique étant choisi parmi l'acétone, le tétrahydrofurane, l'éthanol, le méthanol, le dichlorométhane et leurs mélanges.

**10.** Procédé selon l'une quelconque des revendications précédentes, la solution aqueuse tamponnée ayant un pH de 5 à 8, ou de 5 à 7.

**11.** Procédé selon l'une quelconque des revendications précédentes, la solution aqueuse tamponnée et/ou la solution organique comprenant en outre un tensioactif ; facultativement, le tensioactif étant choisi parmi les tensioactifs ioniques ou non ioniques pharmaceutiquement acceptables, les tensioactifs ioniques comprenant, sans s'y limiter, les phospholipides et le dodécylsulfate de sodium, et les tensioactifs non ioniques comprenant, sans s'y limiter, les polysorbates et les poloxamères.

**12.** Procédé selon l'une quelconque des revendications précédentes, à l'étape c) le rapport du volume de la solution aqueuse tamponnée au volume de la solution organique étant d'au moins 1, et de préférence de 2 à 20, ou de 3 à 10, respectivement.

**13.** Procédé selon l'une quelconque des revendications précédentes, la solution organique et la solution aqueuse entrant en collision sous forme de jets en collision à une vitesse élevée supérieure à 1 m/s, le nombre de Reynold étant supérieur à 500 ; ou la vitesse étant supérieure à 50 m/s, ladite vitesse étant la vitesse de chacun des flux en collision.

**14.** Procédé selon l'une quelconque des revendications précédentes, la solution organique et la solution aqueuse étant pulvérisées à travers des buses inférieures à 1000 $\mu$m, ou inférieures à 500 $\mu$m, ou inférieures à 300 $\mu$m, à des pressions supérieures à 1 bar environ, ou éventuellement supérieure à 10 bars environ, ou supérieure à 50 bars environ.

**15.** Procédé selon l'une quelconque des revendications précédentes, les temps de mélange des deux flux étant inférieurs à 1 milliseconde environ, de préférence inférieurs à 0,5 milliseconde environ, ou inférieurs à 0,1 milliseconde environ.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014043208 A1
- EP 1165224 A

- WO 0061275 A2

**Non-patent literature cited in the description**

- **MAKATO UYAMA et al.** *Langmuir,* 2009, vol. 25 (8), 4336-4338
- **IRIS DUARTE et al.** *European Journal of Pharmaceutical Sciences,* 2016, vol. 93 (10), 203-214
- Hypromellose Acetate Succinate. *United States Pharmacopia and National Formulary, NF 29,* 1548-1550

- BCS: Guidance for Industry: Waiver of In Vivo Bioavailability and Bioequivalence Studies for Immediate-Release Solid Oral Dosage Forms Based on a Biopharmaceutics Classification System. *FDA,* December 2017
- **LESLIE Z. BENET.** Predicting Drug Disposition via Application of a Biopharmaceutics Drug Disposition Classification System. *Basic Clin Pharmacol Toxicol.,* March 2010, vol. 106 (3), 162-167